# FASCICULE DE BREVET EUROPEEN

(11) **EP 1 123 056 B1**
(45) Date de publication et mention de la délivrance du brevet: **21.07.2004**
(21) Numéro de dépôt: 99949075.8
(22) Date de dépôt: 19.10.1999
(51) Int. Cl.: A61B 17/70

(54) **PLAQUE ANTERIEURE POUR VERTEBRES LOMBAIRES ET INSTRUMENT POUR SON POSITIONNEMENT**
VORDERSEITENPLATTE FÜR LENDENWIRBEL UND POSITIONIERWERKZEUG DAFÜR
ANTERIOR OSTEOSYNTHESIS PLATE FOR LUMBAR VERTEBRAE OR SACRAL LUMBAR VERTEBRA AND INSTRUMENT FOR POSITIONING SAME

(30) Priorité: 19.10.1998 FR 9813225
(43) Date de publication de la demande: 16.08.2001
(73) Titulaire: SCIENT'X, 75007 Paris (FR)
(72) Inventeur: JACKOWSKI, André, Birmingham, Warwickshire B31 2HP (GB); FIERE, Vincent, F-69006 Lyon (FR); BILLOT, Charles, F-95160 Montmorency (FR); LEMAITRE, Philippe, F-94140 Alfortville (FR)
(74) Mandataire: Thibault, Jean-Marc
(86) Numéro de dépôt international: PCT/FR1999/002543
(87) Numéro de publication internationale: WO 2000/022999

(56) Documents cités:
- US-A- 5 603 713
- US-A- 5 616 144
- H.S.AN ET J.M.COTLER (EDS.): "Spinal Instrumentation" 1992 , WILLIAMS & WILKINS XP002108102 22396 page 379 -page 396

## Description

### DOMAINE TECHNIQUE :

L'objet de l'invention concerne le domaine technique général des dispositifs de liaison intervertébrale conçus pour corriger un affaiblissement des vertèbres ou un défaut de posture de la colonne vertébrale par stabilisation autour de l'axe longitudinal et selon le plan antéro-postérieur.

L'objet de l'invention concerne, plus précisément, les dispositifs de liaison pour les vertèbres lombaires ou les vertèbres lombaire-sacrée, destinés à être fixés sur la face antérieure desdites vertèbres.

### TECHNIQUE ANTERIEURE :

Il est connu dans l'état de la technique de nombreux types de dispositifs de liaison intervertébrale. Ainsi, il est connu, notamment, par le dépôt de modèle français n° **96 7390**, une plaque d'ostéosynthèse antérieure pour vertèbres lombaires comportant un corps allongé adapté pour recouvrir au moins partiellement la partie antérieure de deux vertèbres consécutives. Ce corps allongé possède une face antérieure et une face postérieure opposée destinée à être appliquée contre la face antérieure des vertèbres. Ce corps allongé présente des zones d'extrémités reliées entre elles par une zone de liaison et conformées chacune sous la forme d'un crochet. Chaque zone d'extrémité est pourvue d'un trou de passage pour une vis destinée à être ancrée dans le corps des vertèbres.

Une telle plaque permet de solidariser les vertèbres entre elles après le rétablissement de l'espace intervertébral. Une telle plaque qui évite la rotation des vertèbres autour de l'axe longitudinal et leur basculement dans le plan antéro-postérieur, permet de stabiliser les vertèbres en favorisant leur arthrodèse.

La plaque antérieure lombaire décrite ci-dessus présente un certain nombre d'inconvénients. Ainsi, il apparaît que chaque partie terminale de la plaque en forme de crochet est destinée à venir s'ancrer sur les bords antérieurs des plateaux des corps des vertèbres. Or, il doit être considéré que d'un patient à un autre, d'une part, les bords antérieurs des plateaux présentent des profils variables et, d'autre part, les dimensions de l'espace intervertébral varient. Il s'ensuit qu'il apparaît difficile de positionner correctement une telle plaque même en disposant d'un jeu de plaques présentant des dimensions et des profils différents. Mis à part l'obligation de disposer d'une gamme importante de plaques, il doit être constaté que le mauvais positionnement d'une telle plaque affecte la qualité de la liaison intervertébrale et, par suite, la stabilité intervertébrale pendant la fusion osseuse.

Une plaque selon le préambule de la revendication 1 est connu de US-A-5 603 713.

### EXPOSE DE L'INVENTION :

L'objet de l'invention vise à remédier aux inconvénients énoncés ci-dessus en proposant une plaque d'ostéosynthèse antérieure pour vertèbres lombaires ou lombaire-sacrée, conçue pour présenter une grande adaptabilité de montage, indépendamment des dimensions présentées par l'espace intervertébral et des dimensions ou/et du profil du corps vertébral sur lequel une telle plaque est destinée à être fixée.

Pour atteindre cet objectif, l'invention propose une plaque d'ostéosynthèse antérieure pour vertèbres lombaires ou lombaire-sacrée du type comportant :
- un corps allongé :
   . adapté pour recouvrir, au moins partiellement, la partie antérieure de deux vertèbres consécutives,
   . possédant une face antérieure et une face postérieure opposée destinée à être appliquée contre la face antérieure des vertèbres,
   . présentant des zones d'extrémités reliées entre elles par une zone de liaison,
   . et muni de deux trous de passage pour des vis d'ancrage, ménagés chacun dans une zone d'extrémité,
la face postérieure du corps de la plaque présente en relation de chaque zone d'extrémité, un plateau d'appui destiné à venir en contact avec la face antérieure des vertèbres.

Selon l'invention, les plateaux d'appui sont, d'une part, inclinés dans le plan sagittal pour définir entre-eux un angle obtus adapté à l'angulation relative de la partie antérieure des deux vertèbres et, d'autre part, reliés entre-eux au niveau de la zone de liaison, par un prolongement concave, de sorte qu'au moins la face postérieure du corps présente un profil concave dans le plan sagittal.

Selon une autre caractéristique de l'invention, cette plaque comporte, en tant que trous de passage, des alésages aménagés de manière que leurs axes de symétrie définissent chacun dans le plan sagittal, un angle d'inclinaison donné par rapport au plan d'extension du plateau d'appui associé, en vue de diverger entre-eux à partir du corps.

Une telle caractéristique permet d'obtenir un ancrage des vis selon une direction divergente permettant une reprise des efforts susceptibles de s'exercer sur la plaque.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

### BREVE DESCRIPTION DES DESSINS :

La **fig. 1** est une vue schématique d'un exemple de montage d'une plaque d'ostéosynthèse antérieure selon l'invention, pour vertèbres lombaires.
La **fig. 2** est une vue de 3/4 avant d'une plaque selon une première variante de réalisation illustrée à la **fig. 1** pour vertèbres lombaires.
La **fig. 3** est une vue 3/4 arrière d'une plaque antérieure selon la première variante de réalisation.
La fig. 4 est une vue de face d'une plaque conforme à la première variante de réalisation.
La **fig. 5** est une vue en coupe-élévation prise sensiblement selon les lignes X-X de la fig. 4.
La **fig. 6** est une vue en élévation d'un instrument permettant la mise en place d'une plaque conforme à l'invention.
La **fig. 7** est une vue de dessus de l'instrument illustré à la **fig. 6.**
La **fig. 8** est une vue en perspective montrant un détail caractéristique de réalisation de l'instrument.
La **fig. 9** est une vue schématique montrant une deuxième variante de réalisation d'une plaque antérieure de liaison entre les vertèbres lombaire-sacrée.
La **fig. 10** est une vue 3/4 avant d'une plaque conforme à la deuxième variante de réalisation.
La **fig. 11** est une vue 3/4 arrière d'une plaque conforme à la deuxième variante de réalisation.
La **fig. 12** est une vue de face d'une plaque conforme à la deuxième variante de réalisation.
La **fig. 13** est une vue en coupe-élévation prise sensiblement selon les lignes X-X de la **fig. 12.**

### MEILLEURE MANIERE DE REALISER L'INVENTION :

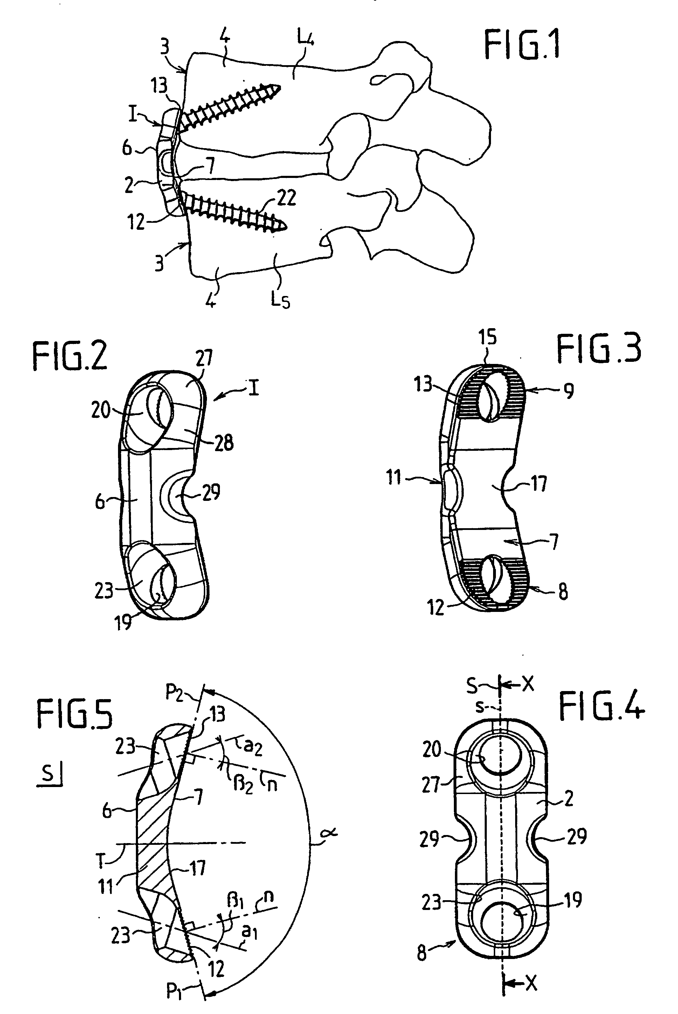

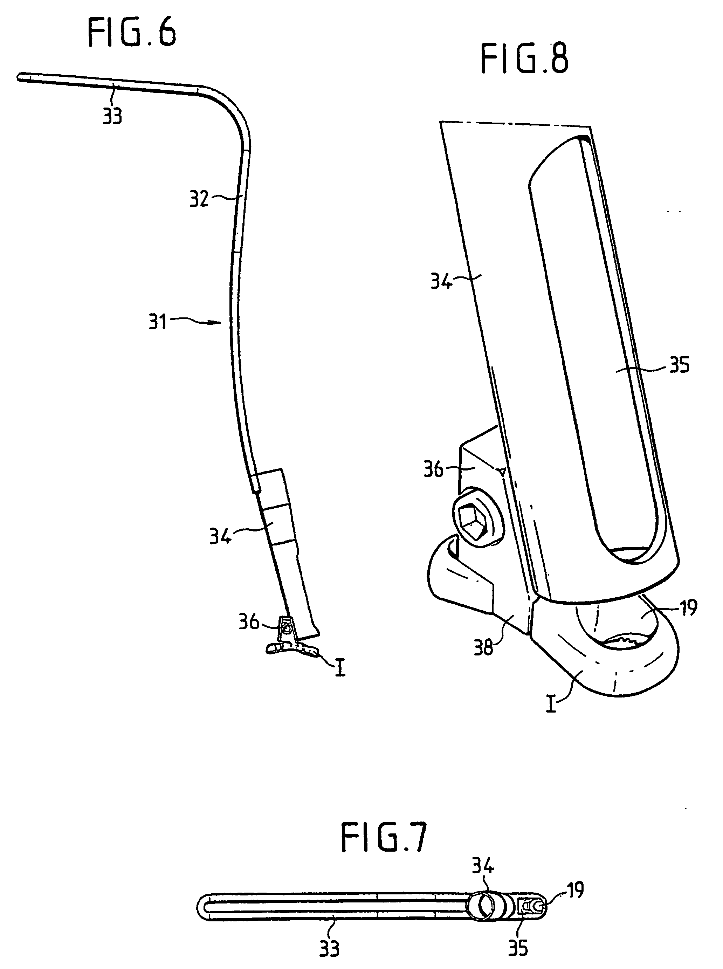

Les **fig. 1** à **5** illustrent une première variante de réalisation d'une plaque d'ostéosynthèse antérieure **I** destinée à assurer l'immobilisation relative entre deux vertèbres lombaires, à savoir par exemple **L**_{**4**}**, L**_{**5**}. La plaque **I** selon l'invention se présente sous la forme d'un corps allongé **2** de forme sensiblement parallélèpipédique possédant un axe longitudinal s par lequel passe un plan de symétrie longitudinal **S** confondu avec le plan sagittal de la plaque (**fig. 4**). La longueur du corps **2** est adaptée pour couvrir au moins partiellement la partie ou la face antérieure **3** du corps **4** des deux vertèbres consécutives à savoir **L**_{**4**}**, L**_{**5**} dans l'exemple illustré.

Le corps **2** comporte une face antérieure **6** et une face postérieure **7** opposée à la face antérieure **6.** La face postérieure **7** est destinée à être appliquée contre la face antérieure **3** des corps vertébraux **4.** Le corps **2** présente deux zones d'extrémités **8, 9** reliées entre elles par une zone centrale de liaison **11.**

Conformément à l'invention, la face postérieure **7** du corps **2** présente, en relation de chaque zone d'extrémité **8** et **9**, respectivement, un plateau d'appui **12**, **13** destiné à venir en contact avec la face antérieure des vertèbres. Chaque plateau d'appui **12, 13** s'étend dans un plan d'extension respectivement **P**_{**1**} et **P**_{**2**} perpendiculaire au plan sagittal ou de symétrie S de la plaque **2.** A la **fig. 5,** le plan sagittal **S** est considéré comme confondu avec le plan de la feuille. Selon une caractéristique de l'invention, les plateaux d'appui **12** et **13** sont inclinés dans le plan sagittal **S** pour définir entre-eux un angle obtus α. Tel que cela ressort plus précisément de la **fig. 5,** les plans **P**_{**1**} et **P**_{**2**} définissent entre-eux un angle obtus α présentant, selon cette variante de réalisation, une valeur comprise entre 140 et 160° et, de préférence, de l'ordre de 150°. L'angle obtus α possède une valeur adaptée à l'angulation relative formée par la partie antérieure **3** des deux vertèbres **L**_{**4**}**, L**_{**5**}**.** Selon cette variante de réalisation, les plateaux d'appui **P**_{**1**}**, P**_{**2**} s'étendent symétriquement l'un par rapport à l'autre, de sorte que chaque plan d'extension **P**_{**1**}**, P**_{**2**} des plateaux d'appui forment un demi-angle α avec le plan transversal **T** perpendiculaire à l'axe longitudinal s.

Selon une caractéristique préférée de réalisation, chaque plateau d'appui **12, 13** est pourvu, tel que cela ressort plus précisément de la **fig. 3,** d'aspérités **15** favorisant l'ancrage de la plaque **2** sur la partie antérieure **3** des vertèbres. La présence d'aspérités **15** favorise également le montage de la plaque **2** sur la partie antérieure **3** lors de son positionnement sur cette face. Dans l'exemple illustré, les aspérités **15** sont constituées par une succession de nervures aménagées selon une direction perpendiculaire à l'axe de symétrie longitudinal s. Bien entendu, il est clair que les aspérités 15 peuvent présenter divers profils différents de nervures transversales. De préférence encore, les rainures sont aménagées à partir du bord extrême du corps **2** en recouvrant au moins partiellement la surface de chaque plateau d'appui **12**, **13**.

Tel que cela ressort plus précisément des **fig. 3** et **5,** les plateaux d'appui **12,13** sont reliés entre-eux au niveau de la zone de liaison **11,** par un prolongement concave **17,** de sorte qu'au moins la face postérieure **7** du corps présente un profil concave dans le plan sagittal **S**.

Selon une autre caractéristique préférée de réalisation, la plaque **2** comporte des trous de passage **19** et **20** réalisés respectivement, chacun dans une zone d'extrémité **8** et **9**. Les trous de passage 19, 20 débouchent sur la face antérieure 6 et sur la face postérieure 7 au niveau des plateaux d'appui **12**, **13.** Selon une caractéristique avantageuse de l'invention, les trous de passage **19** et **20** constituent des alésages aménagés de manière que leurs axes **a**_{**1**}**, a**_{**2**} définissent chacun dans le plan sagittal **S,** un angle d'inclinaison β₁ et β₂ par rapport à la normale n au plan d'extension **P**_{**1**}**, P**_{**2**} du plateau d'appui associé, en vue de diverger entre-eux à partir du corps **2**. Selon la première variante de réalisation, les angles β₁ et β₂ présentent des valeurs identiques. De préférence, chaque alésage **19, 20** présente dans le plan sagittal **S**, un angle d'inclinaison β₁, β₂ dont la valeur est comprise entre 28 et 38° et, de préférence, de l'ordre de 33°.

Les alésages **19**, **20** permettent le passage et le positionnement de vis d'ancrage **22** qui en position d'introduction à l'intérieur des alésages, se trouvent centrées selon les axes **a**_{**1**} et **a**_{**2**}. Selon une caractéristique préférée de réalisation, chaque alésage **19, 20** débouche sur la face antérieure **6** du corps par l'intermédiaire d'un logement **23** de positionnement de la tête d'une vis d'ancrage. Bien entendu, chaque logement **23** présente une section d'ouverture adaptée pour permettre l'introduction des vis d'ancrage à partir de la face antérieure **6** selon la direction définie par les axes **a**_{**1**} et **a**_{**2**} des alésages.

Selon une caractéristique préférée de réalisation, le corps **2** présente, dans le plan sagittal **S**, et au moins au niveau de l'axe de symétrie longitudinal **s**, une épaisseur sensiblement constante. En d'autres termes et tel que cela apparaît plus précisément à la **fig. 5**, le corps **2** présente une face antérieure **6** qui présente un profil général concave sensiblement analogue au profil de la face postérieure **7**. D'une manière plus précise, la face antérieure **6** comprend, au niveau de la partie de liaison **11**, une face plane qui s'étend jusqu'aux bords des logements **23**. Au niveau des logements **23**, la face antérieure 6 présente un profil sensiblement parallèle aux plateaux d'appui **12**, **13**. Selon une variante préférée de réalisation, la face antérieure 6 présente sur toute sa périphérie, un prolongement convexe **27** se raccordant aux faces latérales **28** du corps reliant les faces antérieure **6** et postérieure **7** entre elles. De préférence, les faces latérales **28** sont reliées entre elles par des congés arrondis. Il s'ensuit que le corps **2** présente, à partir de sa face antérieure **6** jusqu'au niveau de sa face postérieure 7, des formes arrondies dépourvues d'aspérités ou d'angles saillants. Un tel profil arrondi du corps **2** permet d'éviter un risque de lésion ou de blessure, en particulier, pour les liaisons nerveuses, les vaisseaux ou les tissus situés à proximité.

Selon une caractéristique préférée de réalisation, le corps **2** présente, sur ses faces latérales opposées **28** et au niveau de la zone de liaison **11,** deux échancrures **29** destinées à coopérer avec un instrument de positionnement de la plaque.

Les **fig. 6** à **8** illustrent, à titre d'exemple, un instrument **31** permettant le positionnement d'une plaque d'ostéosynthèse **I** conforme à l'invention. Un tel instrument **31** comporte un manche **32** conformé pour présenter, selon une direction sensiblement perpendiculaire à son sens d'extension principal, une poignée de préhension **33**. Le manche **32** est pourvu à l'extrémité opposée de celle formant la poignée **33**, d'un tube de guidage **34** pour des outils non représentés mais connus en soi et assurant les diverses opérations pour l'ancrage des vis **22**. Le tube **34** est pourvu, à sa partie inférieure et selon une longueur axiale déterminée, d'une lumière de visée **35** s'établissant sur la partie frontale du tube **34** par rapport à la poignée **33** de l'instrument. Le tube **34** comporte également, à sa partie inférieure et de façon diamètralement opposée à la lumière **35,** un sabot **36** de préhension d'une plaque **I**. Tel que cela apparaît plus précisément aux fig. **7** et **8,** en position de préhension d'une plaque **I**, la projection de la lumière longitudinale de visée **35** coïncide au moins partiellement, avec la section de passage inférieure du tube de guidage qui est destinée à venir s'étendre concentriquement avec un alésage **19, 20** du corps de la plaque **I** maintenue par le sabot **36**. De préférence, le sabot **36** maintient la plaque **I** en position par l'intermédiaire de deux ergots 38 destinés à s'engager par déformation élastique dans les échancrures **29** de la plaque **I**. Il est à noter que la plaque **I** est maintenue dans une position stable de référence, par le sabot **36,** dans la mesure où l'extrémité opposée de celle se trouvant en superposition du tube **34,** est en appui contre le sabot **36.**

La mise en oeuvre de la plaque d'ostéosynthèse selon l'invention découle directement de la description qui précède.

Préalablement au montage de la plaque sur les vertèbres d'un patient, les parties de la face antérieure **3** des vertèbres devant recevoir les plateaux d'appui **12, 13,** sont préparées pour présenter sur chaque corps vertébral un méplat. Pour sa mise en place sur les vertèbres ainsi préparées, la plaque **I** est montée par encliquetage sur le sabot de guidage **38** de l'instrument **31**. Grâce à la présence de la lumière longitudinale **35** dont la projection coïncide avec un alésage **19, 20** pratiqué dans la plaque, l'opérateur peut visualiser les opérations classiques de mise en place des vis 22 sur les vertèbres. Il est à noter que pendant ces opérations, les outils se trouvent guidés en déplacement par le tube **34**. A cet égard, l'angulation relative entre le tube de guidage **34** et le sabot de préhension **36** est choisie de manière que l'axe du tube **34** coïncide avec l'axe **a**_{**1**}**, a**_{**2**} d'un alésage, lorsque la plaque **2** occupe sa position d'appui stable contre le sabot. Il s'ensuit un positionnement correct des vis d'ancrage **22** qui se trouvent parfaitement guidées par l'intermédiaire des alésages orientés **19, 20.** Il doit être considéré que la mise en place de la plaque **I** est facilitée par la présence des aspérités **15** empêchant le glissement de la plaque **I**.

Après la mise en place d'une vis d'ancrage **22**, l'instrument **31** est désolidarisé de la plaque **2** par une simple traction et, après retournement de 180°, est encliqueté sur la plaque, afin d'autoriser l'exécution des opérations de mise en place de l'autre vis d'ancrage **22**.

Tel que cela ressort de la **fig. 1,** la plaque **1** se trouve, après montage, en contact par ses plateaux d'appui **12, 13** sur les méplats des corps vertébraux **4**. Il s'ensuit un contact plan entre chaque plateau d'appui **12, 13** et la face antérieure **3** du corps vertébral **4**. La qualité d'un tel contact associé au montage divergent des vis d'ancrage **22** permettent d'obtenir une reprise des efforts et une stabilisation des vertèbres entre elles. Il est à noter que la zone de liaison **11** de la plaque présente un prolongement concave **17** qui se trouve situé en vis-à-vis de l'espace intervertébral. Le dégagement présenté à cet endroit par la plaque **I** permet de s'adapter aux différentes formes des espaces intervertébraux rencontrés.

### POSSIBILITE D'APPLICATION INDUSTRIELLE :

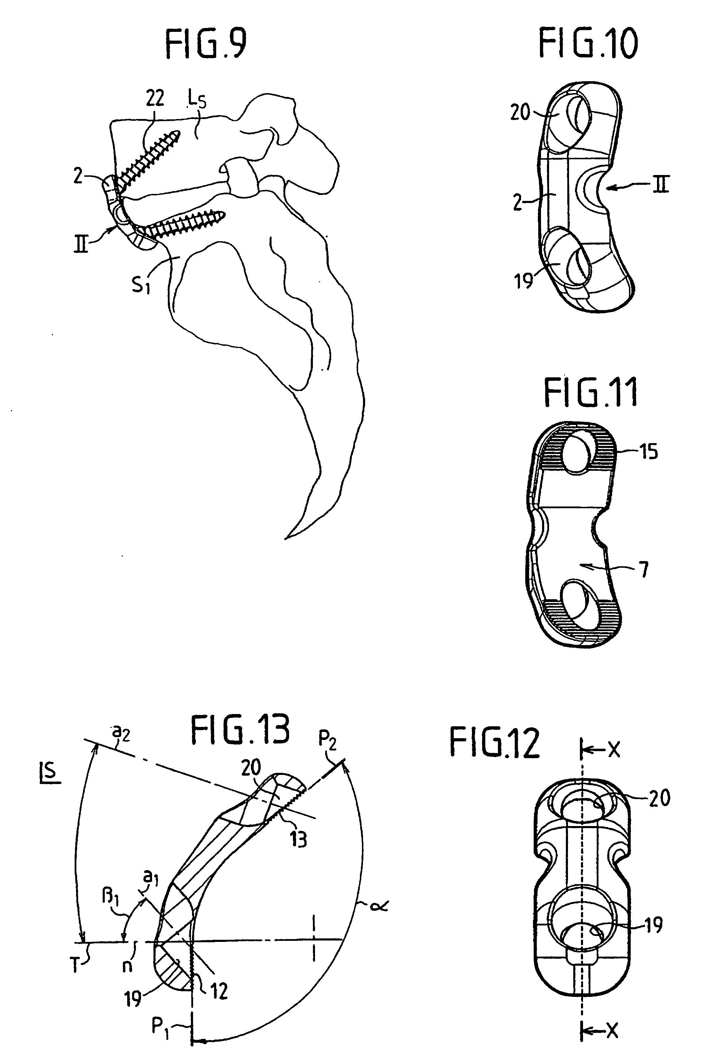

Dans l'exemple de réalisation décrit ci-dessus, la plaque d'ostéosynthèse est particulièrement adaptée pour solidariser entre elles deux vertèbres lombaires. Bien entendu, l'objet de l'invention peut être appliqué à une plaque d'ostéosynthèse entre une vertèbre lombaire et une vertèbre sacrée à savoir **L**_{**5**}**-S**_{**1**}. Les fig. **9** à **13** illustrent une deuxième variante de réalisation d'une plaque d'ostéosynthèse **II** conforme à l'invention adaptée pour la solidarisation des vertèbres lombaire et sacrée.

Il est à noter que les éléments communs de cette plaque **II** avec ceux décrits en relation de la première variante de réalisation conservent les mêmes références. Selon cette seconde variante de réalisation, les plateaux d'appui **12, 13** de la plaque ne sont pas symétriques l'un par rapport à l'autre selon l'axe transversal **T**. Tel que cela ressort plus précisément de la **fig. 13**, les deux plateaux d'appui **12, 13** sont inclinés entre-eux selon le plan sagittal S pour définir un angle α adapté à l'angulation relative de la partie antérieure des deux vertèbres **L**_{**5**}**-S**_{**1**}. L'angle obtus α est compris entre 120 et 140° et, de préférence, de l'ordre de 130°. De plus, l'alésage **19** présente dans le plan sagittal, un angle d'inclinaison β₁ compris entre 45 et 55° et, de préférence, de l'ordre de 50°, considéré entre la normale **n** au plan **P**_{**1**} et l'axe **a**_{**1**} de l'alésage **19**. Dans le même sens, l'alésage **20** présente dans le plan sagittal un angle d'inclinaison β₂ compris entre 15 et 25° et, de préférence, de l'ordre de 20°, considéré entre la normale n au plateau d'appui **12** et l'axe **a**_{**2**} de l'alésage **20**.

L'invention n'est pas limitée aux exemples décrits et représentés, car diverses modifications peuvent y être apportées sans sortir de son cadre.

## Revendications

1. Plaque d'ostéosynthèse antérieure pour vertèbres lombaires ou lombaire-sacrée, du type comportant :
- un corps allongé (**2**) :
. adapté pour recouvrir, au moins partiellement, la partie antérieure de deux vertèbres consécutives,
. possédant une face antérieure (**6**) et une face postérieure (**7**) opposée destinée à être appliquée contre la face antérieure (**3**) des vertèbres,
. présentant des zones d'extrémités (**8**, **9**) reliées entre-elles par une zone de liaison (**11**),
. et muni de deux trous de passage (**19**, **20**) pour des vis d'ancrage (**22**), ménagés chacun dans une zone d'extrémité,
la face postérieure (7) du corps (**2**) de la plaque présentant en relation de chaque zone d'extrémité, un plateau d'appui (**12**, **13**) destiné à venir en contact avec la face antérieure (**3**) des vertèbres, **caractérisée en ce que** les plateaux d'appui (**12**, **13**) sont, d'une part, inclinés pour définir entre-eux, selon le plan sagittal (**S**), un angle obtus (α) adapté à l'angulation relative de la partie antérieure des deux vertèbres et, d'autre part, reliés entre-eux au niveau de la zone de liaison, par un prolongement concave (**17**), de sorte qu'au moins la face postérieure (7) du corps présente un profil concave dans le plan sagittal.

2. Plaque selon la revendication 1, **caractérisée en ce que** chaque plateau d'appui (**12**, **13**) est pourvu d'aspérités (**15**) pour favoriser l'ancrage de la plaque sur la partie antérieure (**3**) des vertèbres.

3. Plaque selon la revendication 1, **caractérisée en ce qu'**elle comporte en tant que trous de passage (**19**, **20**), des alésages aménagés de manière que leurs axes (**a**_{**1**}**, a**_{**2**}) définissent chacun dans le plan sagittal, un angle d'inclinaison (β₁, β₂) donné par rapport au plan d'extension (**P**_{**1**}**, P**_{**2**}) du plateau d'appui associé, en vue de diverger entre-eux à partir du corps.

4. Plaque selon la revendication 3, **caractérisée en ce qu'**elle comporte deux alésages (**19**, **20**) présentant chacun dans le plan sagittal (**S**), un angle d'inclinaison (β₁, β₂) de l'ordre de 33° par rapport à la normale (**n**) au plan d'extension du plateau d'appui associé (**P**_{**1**}**, P**_{**2**}).

5. Plaque selon la revendication 3, **caractérisée en ce qu'**elle comporte deux alésages (**19, 20**) présentant dans le plan sagittal (**S**), des angles d'inclinaison (β₁, β₂) respectivement de l'ordre de 50° et 20° par rapport à la normale (**n**) au plan d'extension d'un plateau d'appui.

6. Plaque selon la revendication 4, **caractérisée en ce que** les plateaux d'appui (**12**, **13**) s'étendent symétriquement l'un par rapport à l'autre en définissant entre-eux un angle obtus (α) de l'ordre de 150°.

7. Plaque selon la revendication 5, **caractérisée en ce que** les plateaux d'appui (**12, 13**) se trouvent décalés entre-eux selon le plan sagittal d'un angle obtus (α) de l'ordre de 130°.

8. Plaque selon la revendication 3, **caractérisée en ce que** chaque alésage (**19**, **20**) débouche sur la face antérieure (**6**) du corps par l'intermédiaire d'un logement (**23**) de positionnement de la tête d'une vis d'ancrage.

9. Plaque selon l'une des revendications 1 à 8, **caractérisée en ce que** le corps (**2**) comporte dans sa zone de liaison (**11**), deux échancrures (**29**) destinées à coopérer avec un instrument (**31**) de positionnement de la plaque.

10. Plaque selon l'une des revendications 1 à 9, **caractérisée en ce que** le corps (**2**) présente au moins dans le plan sagittal (**S**), une épaisseur sensiblement constante.

11. Plaque selon la revendication 10, **caractérisée en ce que** le corps présente une face antérieure (**6**) raccordée par un prolongement convexe (**27**) à des faces latérales arrondies (**28**) reliées à la face postérieure (**7**).

12. Instrument pour le positionnement d'une plaque d'ostéosynthèse (**I, II**) conforme à l'une des revendications 1 à 11, **caractérisé en ce qu'**il comporte un manche (**32**) pourvu d'un tube de guidage (**34**) pour un outil, le tube (**34**) étant pourvu, à sa partie inférieure et de façon diamétralement opposée, d'une part, d'un sabot de préhension (**36**) d'une plaque et, d'autre part, d'une lumière longitudinale de visée (**35**), dont la projection coïncide au moins partiellement avec la section de passage inférieure du tube de guidage, qui est destinée à venir s'étendre concentriquement avec un alésage (**19**, **20**) du corps (**2**) maintenu par le sabot (**36**).

## Claims

1. Anterior osteosynthesis plate for lumbar or sacral lumbar vertebrae of the type comprising:
- an elongated body (2)
. adapted to cover, at least in part, the anterior portion of two consecutive vertebrae,
. having an anterior face (6) and opposite posterior face (7) intended to be applied against the anterior face (3) of the vertebrae,
. having end zones (8, 9) connected together by a link zone (11),
. and provided with two passage holes (19, 20) for anchor screws (22) each arranged in an end zone,
the posterior face (7) of plate body (2) in relation with each end zone having a bearer plateau (12, 13) intended to come into contact with the anterior face (3) of the vertebrae, **characterized in that** the bearer plateaux (12, 13) are firstly inclined to define between themselves and along a sagittal plane (S) an obtuse angle (α) adapted to the relative angle of the anterior portion of the two vertebrae, and secondly are joined together at the link zone by a concave extension (17) so that at least the posterior face (7) of the body has a concave profile in the sagittal plane.

2. Plate as in claim 1, **characterized in that** each bearer plateau (12, 13) has a roughened surface(15) to promote anchoring of the plate onto the anterior portion (3) of the vertebrae.

3. Plate as in claim 1, **characterized in that** as passage holes (19, 20) it comprises bore holes made so that their axes (a₁, a₂) along the sagittal plane each define a given angle of inclination (β₁, β₂)relative to the plane of extension (P₁, P₂) of the associated bearer plateau, so that they diverge from one another from the body.

4. Plate as in claim 3, **characterized in that** it comprises two bore holes (19, 20) each having in the sagittal plane (S) an angle of inclination (β₁, β₂)in the order of 33° relative to the normal (n) to the plane of extension of the associated bearer plateau (P₁, P₂).

5. Plate as in claim 3, **characterized in that** it comprises two bore holes (19, 20) having angles of inclination (β₁, β₂) in the sagittal plane (S) of the order of 50° and 20° respectively relative to the normal (n) to the plane of extension of a bearer plateau.

6. Plate as in claim 4 **characterized in that** the bearer plateaux (12, 13) extend symmetrically relative to one another defining between them an obtuse angle (α) of the order of 150°C.

7. Plate as in claim 5, **characterized in that** the bearer plateaux (12, 13) are offset in the sagittal plane by an obtuse angle (α) of the order of 130°.

8. Plate as in claim 3, **characterized in that** each bore hole (19, 20) leads to the anterior face (6) of the body via a housing (23) for positioning the head of an anchor screw.

9. Plate as in any of claims 1 to 8, **characterized in that** the body (2) in its link zone (11) comprises two notches (29) intended to cooperate with a plate positioning instrument (31).

10. Plate as in any of claims 1 to 9, **characterized in that** the body (2) at least in the sagittal plane (S) is of substantially constant thickness.

11. Plate as in claim 10, **characterized in that** the body has an anterior face (6) connected via a convex extension (27) to rounded lateral faces (28) joined to the posterior face (7).

12. Instrument for positioning an osteosynthesis plate (I, II) according to any of claims 1 to 11, **characterized in that** it comprises a handle (32) provided with a guide tube (34) for an implement, the tube (34) being provided at its lower part, and in diametrically opposite fashion, firstly with a plate grasping shoe (36) and secondly with a longitudinal sighting port (35) whose projection coincides at least partly with the lower passage section of the guide tube, intended to extend concentrically with a bore hole (19, 20) of body (2) held by shoe (36).

## Patentansprüche

1. Vorderseiten-Osteosyntheseplatte für Lendenwirbel oder Lenden-Beckenwirbel des Typs umfassend:
- einem länglichen Körper (2):
• geeignet zum zumindest teilweisen Abdecken des vorderen Teils zweier aufeinander folgender Wirbel,
• mit einer Vorderseite (6) und einer gegenüberliegenden Rückseite (7), die dazu bestimmt ist, gegen die Vorderseite (3) der Wirbel angelegt zu werden,
• mit Endzonen (8, 9), die untereinander durch eine Verbindungszone (11) verbunden sind,
• und mit zwei Durchgangsbohrungen (19, 20) für Verankerungsschrauben (22), die jeweils in einer Endzone eingerichtet sind, versehen,
wobei die Vorderseite (7) des Körpers (2) der Platte in Bezug auf jede Endzone eine Auflageplatte (12, 13) aufweist, die dazu bestimmt ist, mit der Vorderseite (3) der Wirbel in Berührung zu kommen, **dadurch gekennzeichnet, dass** die Auflageplatten (12, 13) einerseits geneigt sind, um untereinander gemäß der Sagittalebene (S) einen stumpfen Winkel (α) festzulegen, der für die relative Anwinkelung des Vorderteils der zwei Wirbel geeignet ist, und andererseits, die untereinander auf der Ebene der Verbindungszone durch eine konkave Verlängerung (17) verbunden sind, so dass zumindest die Rückseite (7) des Körpers ein konkaves Profil in der Sagittalebene aufweist.

2. Platte nach Anspruch 1, **dadurch gekennzeichnet, dass** jede Auflageplatte (12, 13) mit Rauhigkeiten (15) versehen ist, um das Verankern der Platte auf dem Vorderteil (3) der Wirbel zu begünstigen.

3. Platte nach Anspruch 1, **dadurch gekennzeichnet, dass** sie als Durchgangsbohrungen (19, 20) Bohrungen umfasst, die so eingerichtet sind, dass ihre Achsen (a₁, a₂) jeweils in der Sagittalebene in Bezug auf die Erweiterungsebene (P₁, P₂) der zugehörigen Auflageplatte einen gegebenen Neigungswinkel (β₁, β₂) definieren, um ausgehend vom Körper untereinander zu divergieren.

4. Platte nach Anspruch 3, **dadurch gekennzeichnet, dass** sie zwei Bohrungen (19, 20) aufweist, die jeweils in der Sagittalebene (S) in Bezug auf die Senkrechte (n) zur Erweiterungsebene der zugehörigen Auflageplatte (P₁, P₂) einen Neigungswinkel (β₁, β₂) in der Größenordnung von 33° aufweisen.

5. Platte nach Anspruch 3, **dadurch gekennzeichnet, dass** sie zwei Bohrungen (19, 20) aufweist, die in der Sagittalebene (S) in Bezug auf die Senkrechte (n) zur Erweiterungsebene einer Auflageplatte Neigungswinkel (β₁, β₂) jeweils in der Größenordnung von 50° und 20° aufweisen.

6. Platte nach Anspruch 4, **dadurch gekennzeichnet, dass** sich die Auflageplatten (12, 13) symmetrisch zueinander erstrecken und dabei untereinander einen stumpfen Winkel (α) in der Größenordnung von 150° bilden.

7. Platte nach Anspruch 5, **dadurch gekennzeichnet, dass** sich die Auflageplatten (12, 13) in der Sagittalebene untereinander um einen stumpfen Winkel (α) in der Größenordnung von 130° versetzt befinden.

8. Platte nach Anspruch 3, **dadurch gekennzeichnet, dass** jede Bohrung (19, 20) auf der Vorderseite (6) des Körpers über eine Aufnahme (23) zum Positionieren des Kopfs einer Verankerungsschraube mündet.

9. Platte nach einem der Ansprüche 1 bis 8, **dadurch gekennzeichnet, dass** der Körper (2) in seiner Verbindungszone (11) zwei Ausschnitte (29) umfasst, die dazu bestimmt sind, mit einem Instrument (31) zum Positionieren zusammenzuwirken.

10. Platte nach einem der Ansprüche 1 bis 9, **dadurch gekennzeichnet, dass** der Körper (2) zumindest in der Sagittalebene (S) eine im Wesentlichen konstante Dicke aufweist.

11. Platte nach Anspruch 10, **dadurch gekennzeichnet, dass** der Körper eine Vorderseite (6) aufweist, die durch eine konvexe Verlängerung (27) mit abgerundeten Seitenflächen (28) verbunden ist, die mit der Rückseite (7) verbunden sind.

12. Instrument zum Positionieren einer Osteosyntheseplatte (I, II) nach einem der Ansprüche 1 bis 11, **dadurch gekennzeichnet, dass** es einen Stiel (32) umfasst, der mit einem Führungsrohr (34) für ein Werkzeug versehen ist, wobei das Rohr (34) an seinem unteren Teil und diametral gegenüberliegend einerseits mit einem Greifschuh (36) einer Platte und andererseits mit einem Visierlangloch (35) versehen ist, dessen Vorsprung zumindest teilweise mit dem unteren Durchgangsquerschnitt des Führungsrohrs übereinstimmt, das dazu bestimmt ist, sich konzentrisch mit einer Bohrung (19, 20) des Körpers (2), gehalten vom Schuh (36) zu erstrecken.
